# EUROPEAN PATENT SPECIFICATION

(11) **EP 3 371 721 B1**
(45) Date of publication and mention of the grant of the patent: **27.08.2025**
(21) Application number: 15801688.1
(22) Date of filing: 03.11.2015
(51) Int. Cl.: G16H 10/20, G06Q 10/10, G16H 40/67

(54) **METHOD AND SYSTEM TO COLLECT AND HANDLE CLINICAL TRIALS DATA**
METHODE UND SYSTEM ZUR ERFASSUNG UND BEARBEITUNG VON DATEN AUS KLINISCHEN VERSUCHEN
PROCÉDÉ ET SYTÈME DE COLLECTE ET DE TRAITEMENT DE DONNÉES D'ESSAIS CLINIQUES

(43) Date of publication of application: 12.09.2018
(73) Proprietor: Kapadi Italy S.R.L., 20122 Milano (IT)
(72) Inventor: VISANI, Luigi, 52027 San Giovanni Valdarno (IT); MINICH, Peter Josef, 89075 Ulm (DE)
(74) Representative: Giraldi, Elisa
(86) International application number: PCT/EP2015/075549
(87) International publication number: WO 2017/076428

(56) References cited:
- US-A1- 2005 149 852
- US-A1- 2013 239 104
- US-A1- 2016 073 217

## Description

### Field of the Invention

Embodiments of the subject matter disclosed herein relates to a method and a system to collect and handle clinical trials for the benefit of medical practitioners following one or more patients under treatment with a drug under test.

### Background art

The marketing of a drug needs several preliminary test phases to reach the approval of the relative therapy. After biocompatibility tests effected in laboratory, a further and very important test phase comprises the successful overcoming of the so called "clinical trials". They are prospective biomedical or behavioral research studies on human subjects; are designed to answer specific questions about biomedical or behavioral interventions; and are aimed at inferring safety and efficacy data. The decision process of national regulatory authorities for releasing new drugs on the market comprise a thorough analysis of clinical trials results conducted by the pharmaceutical companies. Clinical trials comprise standardized steps to carry out. A multitude of data must be acquired for each step. The data collected must be gathered in the clinical trial file to which refer.

Such data are collected in various way and formats according to the experience of the medical practitioner. Besides his regular duties interacting with subjects under test, he has also the burden to properly take note and store relevant information for his task. In known solutions, case report forms (CRF) are used. A CRF is a paper or electronic questionnaire specifically used in clinical trial research. In practice is a form comprising a plurality of fields that the medical practitioner must fill in after observations on a subject under and/or questions to him.

Document US 2005/149852A1 discloses a method for collecting and handling clinical trials data.

Document US 2013/239104A1 discloses methods to activate a licensed software on a user device.

Documento US 2016/073217A1 discloses methods to verify a license key for carrying out a software method by using an NFC tag.

### Summary of the Invention

The paper version of a CRF requires an amount of extra work for digitalizing data collected by the medical practitioner.

The electronic version of a CRF requires a computer properly configured. Often subjects under test are spread amongst different medical facilities. Often, a suitable technology equipment is not available in each medical facility. So for some subjects, the paper version of CRF must be used, and for some other the electronic one. Such situation impacts on the efficiency of the clinical trial as well as on the quality of data collected.

Furthermore, a clinical trial collects sensible data relating to health status of an individual. The electronic equipment used for generating a CRF may be not secure enough to ensure an adequate level of privacy.

Therefore, there is a need for a method and a system for collecting and handling clinical trials in a simple, safe and direct way.

According to the present invention, there is a method for collecting and handling clinical trials data, the method comprising the following steps:
A - establishing a communication session between said user equipment and management server;
B - at said management server, authenticating said user;
C - at said management server, generating or retrieving one or more case report form associated to said user, said case report form comprising a plurality of information relative to a clinical trial;
D - delivering the one or more case report form generated or retrieved to said user via said user equipment,
E - at said user equipment, updating and/or viewing information of said one or more case report form,
F - at said user equipment, storing said one or more case report updated;
G - at said management server, synchronizing the content of said one or more case report form associated to said user with the content of the ones stored on the user equipment,

Wherein in the step B of authenticating said user comprises a preliminary phase of registration of a user, made once for each user, comprising the following steps:
- at said management server, generating a unique license key,
- record said license number in a NFC tag,
- assigning said NFC tag to said user,
- establishing a communication between said user equipment and said management server,
- at said management server, detecting a unique identifier of said user equipment,
- via said user equipment retrieving the license key stored in said NFC tag,
- transmitting said license number to said management server,
- at said management server registering said user.

The present invention also relates to a system for collecting and handling clinical trials data comprising:
- at least a user equipment associated to a user comprising at least a processing unit, memory means, data communication means, acquiring means and/or proximity communication means,
- at least a management server comprising at least a processing unit, memory means, and data communication means, - a program loaded in said memory means and adapted to be executed by said processing unit, said program when running is configured to carry out a method according to the invention,
- a telecommunication network to which said user equipment and said management server are connected,
- a security device to register said user at said management server.

### Brief description of the drawings

The present invention will become more apparent from the following description of exemplary embodiments to be considered in conjunction with accompanying drawings wherein:
Figure 1 shows a scheme of data configured according the present invention;
Figure 2 shows a block diagram of operations carried out according the present invention;
Figure 3 shows a block diagram of further operations carried out according the present invention;
Figure 4 shows a block diagram representing an embodiment of the life cycle of a computer program product implementing the method according the present invention;
Figure 5 shows a block diagram representing the data processing.
Figure 6 shows a scheme of a system according the present invention;
Figure 7 shows a block diagram representing the architecture of the present invention;
Figure 8 shows a block diagram representing the flows executed in the context of the present invention.

### Detailed Description of the Invention

The same reference numbers in different drawings identify the same or similar elements. The following detailed description does not limit the invention. Instead, the scope of the invention is defined by the appended claims.

According to the invention, there is a method for collecting and handling clinical trials data wherein is provided a user with a user equipment and a management server. Typically the user is a medical practitioner, the user equipment is a smartphone properly configured (as described in the following) or a tablet or customized mobile hardware for the purpose, and the management server is a server known as per se, but properly configured according the present invention (as described in the following).

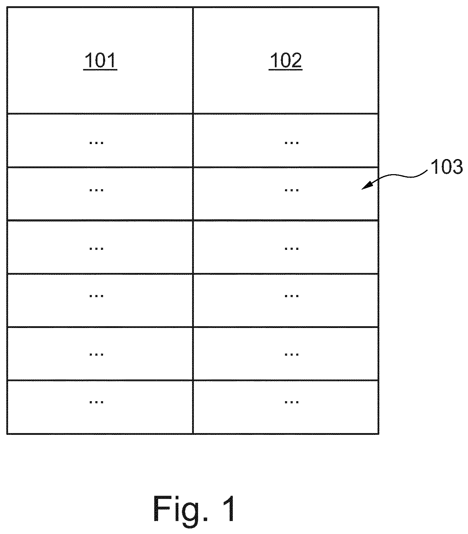

The method comprises the steps of:
- Establishing a communication session between said user equipment and management server. Typically this operation, will occur through connection at the management server through the smartphone. The connection occurs via the Internet, in particular the smartphone is connected to mobile network, where the management server is connected via a fixed network.
- At the management server, the user is authenticated. The user will operate through his smartphone collecting and editing sensible data, it is therefore important asses that the smartphone in object is at the disposal of a user with the rights to operate.
- At the management server, one or more case report form associated to a user are generated or retrieved, according to operation required by the user. Each case report form comprises a plurality of information relative to a clinical trial. Such information may be vital signs under monitoring, site where the survey is effected, etc. In particular, the case report is generally referred to a subject under test and comprise a subset or an extended set of the information described above. Figure 1 shows the logical structure of the solution of a case report form 100. The field 101 contains identifying information of the subject under test. The field 102 represents the medical practitioner (the user in the meaning of the present patent application) conducting the survey on the subject. The fields 103 represents the vital signs under monitoring. The date in which the survey is conducted may be aggregated to the fields 103, as a "time stamp". The following example is given: mister A is the subject under test, doctor B is the medical practitioner conducting the survey, the blood pressure and the heartbeats are the vital signs under monitoring. A first survey is conducted the first June and a second survey is conducted the fifth June. Therefore the field 101 is populated with the value: "Mister A", the field 102 is populated with the value: "doctor B", the fields 103 are populated with the values: "140/65 mmHg_0106"; "65 bpm_0106"; "135/62 mmHg_0506"; "63 bpm_0506". In this example the vital sign detected is associated to the time stamp representing the date in which has been detected.

In general, the management server generates a new case report form the first time a user access the management server and start a new study on a subject under test. Whilst the management server retrieves the case report form associated to the user in subsequent accesses to the management server.
- Delivering the one or more case report form generated or retrieved to the user via said user equipment. The user receives on his user equipment the case report forms generated or the one or more case report forms retrieved. The following scenarios may be described: the user accesses for the first time the management server: he is associated with one or more subjects of the clinical trial. For each subject a case report form is generated, as described above. The user receives on his smartphone the case report forms generated. He may choose the one for which he is going to make his measurements/tests. 2) The user has already done an access to management server, and he wants to retrieve one specific case report form for which he is going to update some information. The management server delivers the case report forms associated to such user. At first the registered study of the user will delivered. After selecting a study the visit list. After selecting a visit a collection of included eCRFs will deliverd. After selecting a eCRF the case report form will delivered and presented in a mobile form. At any of these steps Voice Commands are created for the user navigation. After triggering the App with the initial keyword e.g. "What Ever" graphical overlays will be painted over each navigation field which displays the unique voice command of the navigation field e.g. "open visit 1"-The user choose the one of interest. In one embodiment, the user may choose the case report form directly on the management server, thus the management server delivers the only one of interest for the user.
- At said user equipment, the user updates or views information of the one or more case report forms delivered.

The following possibilities are provided:
The user intends to update information already stored of a case report form. For example, he notes a wrongly transcribed parameter previously measured. It is important to differ if the value / eCRF form is already closed/frozen in the management system. If yes a change can only applied over raising an eQuery. The eQuery stores additional information why an information has changed and when and from whom. If the eCRF is not closed/frozen than an update of the clinicical data can be done anytime. He therefore updates the content of the information in the corresponding case report form.

The user is effecting a further survey on a subject and he has to insert the parameter just measured. The corresponding case report form is retrieved and the relative information are populated correspondingly. According to the present invention, a case report form generated and populated for the first time with corresponding information is considered "updated", the difference resides in that the case report form is not retrieved but newly generated.

The user wants to consult information of one or more subjects viewing their case report forms. The corresponding case report form is delivered and the user may navigate through the relative information with his smartphone.
- At said user equipment, the one or more case report form updated are stored on memory means of such user equipment. In one embodiment, the one or more case report form are stored on the memory of the smartphone. Such operation is important to maintain aligned the case report form updated on the user apparatus with the ones on the management server. This aspect will be clearer from the remaining of the description.
- At said management server, the content of the one or more case report form associated to the user are synchronized with the content of the ones stored on the user equipment. Such operation may be effected according a plurality of techniques. The synchronization occurs in a similar way to a "differential backup". The user via his user equipment sends the case report form updated and stored on its memory. The management server is programmed to check which information of the case report form are different with respect to the ones stored on its memory: such information are updated correspondingly. Such operation is carried out when the user equipment has Internet connectivity, thus a certain amount of time may exist between two subsequent synchronization with the management server. In other words, the information of the case report forms stored on the user equipment may be rather different to the ones on the management server. In this sense, each update on the user equipment may be associated to an indicator that takes trace of the update effected. The management server through indicator may trace back the update effected, and properly synchronizing the information.

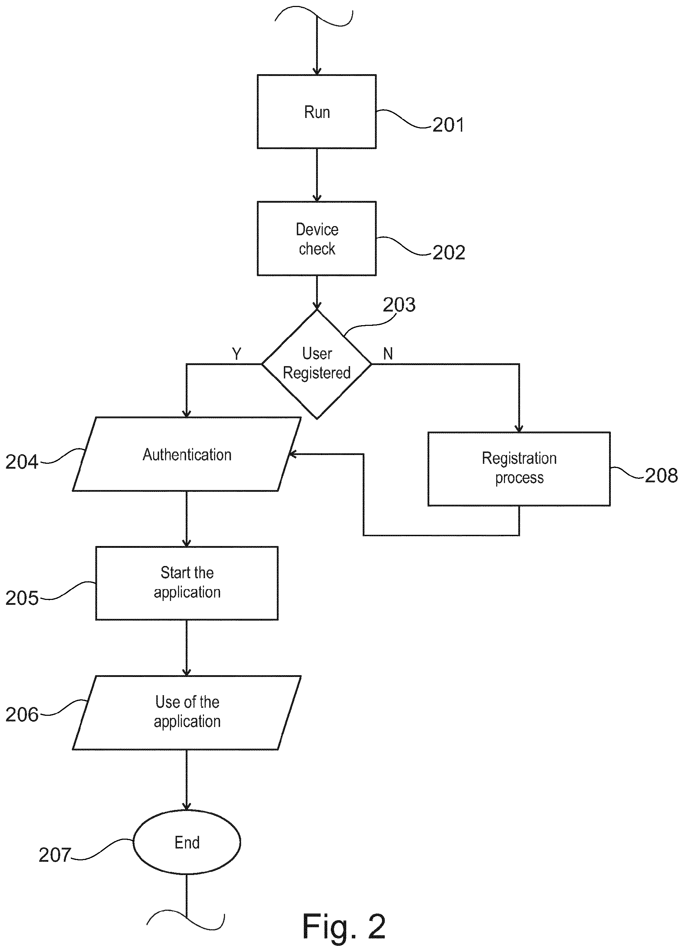

Figure 2 shows a block diagram of the of main steps of a method according the present invention. In particular such diagram is a portion of a more extended diagram representing steps of a method according to the present invention. Such method is implemented as an application for a smartphone.

The block 201 represents the run of the application: the user executes the application loaded into his smartphone. After executing the application, the application carries out a preliminary check of the device. In particular the device is checked if all the components involved in the functioning of the app are correctly working. As an example, according to the application will use the camera of the smartphone to complete the registration process, thus the good functioning of the camera is necessary: the check in the step 202 notifies the user if something does not work properly impairing the completion of the method.

Further checks are internet connectivity, microphone for voice recognition available, management server state

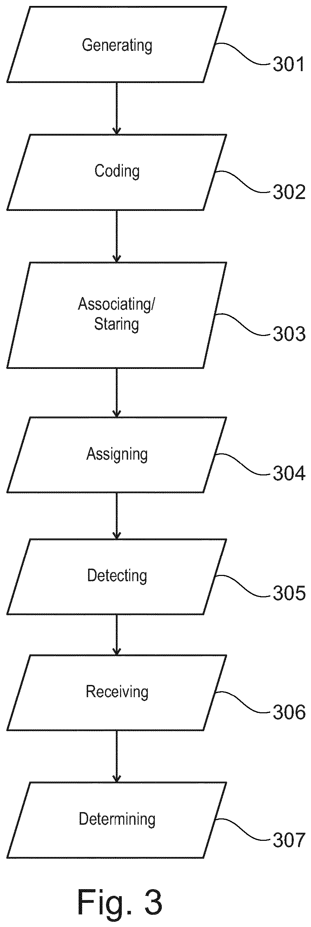

After step 202, there is the test step 203 that checks if a user is already registered at the management server, or such operation has not occurred yet. In case of negative result (the user is not register yet) the line NO is followed to arrive at the block 208, where the registration process occurs. Details of such process are described in detail with reference to figure 3.

After the registration is effected, or after the test 203 is positive (in this case the line YES is followed) the elaboration step 204 is reached. Such step 204 represents the authentication at the management server. In the authentication step is strictly connected to the registration step: for authenticating a user, credentials established during the registration process are checkedIf the authentication step 204 fails, the user will be redirected to the login and will not receive the case report forms associated to him on his smartphone. Other options may be provided as well as a proper exception management logic.

After being authenticated, the step 205 is reached: the user starts using the application. This step is reached receiving the case report form updated on his smartphone, the list of the last case report forms viewed, new case report forms associated to the user, ecc. Depending on the type of management server a user specific encryption of the data transfer will be used. At least SSL for the data transmission is used. The User will be asked here if he want to activate the voice recognition. If he disable the App starts without the Voice Recognition service and the user can use the App by touch and type events.

A symmetry check is carried out on the management server to ensure that the user has the updated case report forms on his smartphone when starts using the application.

At his first execution the user receives a plurality of case report forms associated to him. Such case report forms are partially empty: the data to acquire through the clinical trial are not populated yet.

After effecting clinical trials, the user populates data of the respective case report forms, such data are stored on the smartphone. The user may be out of reach of the Internet network for a long time, this, in principle, does not influence the functioning of the application: the user may keep going acquiring data. In this case, when the application is run at the step 201 the Internet connection is checked. If not available, the application will run in "offline mode" discarding the step 204. The user may keep updating the information of case report form stored. An indicator may take account of the number of updates each case report form had. When the user starts the application and an Internet connection is available, the content stored on the smartphone is aligned with the content on the management server.

New clinical trials for new subjects may be assigned to a user. Starting the application the user receives the new case report forms associated.

After starting the application the user begin to use the application itself. Thus he may:
- Updating case report forms stored on the memory of its smartphone, due to new observations on a subject under test,
- Retrieving case report forms stored to update one or more fields of information,
- Viewing one or more of the case report forms stored on the memory of its smartphone.

After using the application, the user once has finished his observations close the application, as described in the block 207. In one embodiment, such operation finishes off with the commit at the management server of the updated case report form, the management server may thus properly synchronize the content.

Figure 3 shows a block diagrams of operations to register a new user.

As shown in the block 301 at the management server a unique license key is generated. Such key may be a pseudo-random code generated according proper rules. The block 302 is reached wherein the license key is represented as a bidimensional code (a QR code). The QR code typically stores information in form of an encrypted base64 string that represents UIDs of a new potential user in the management server.

At the management server, the QR code is associated to a user, as shown in the block 303.

After associating the code to a user, the step 304 is reached wherein the QR code is physically assigned to a new user.

At the block 305, the user with the QR code establishes a communication with the management server through his user equipment. In this operation, the management server detects a unique code of the smartphone of the user (for example the IMEI code or/and the MAC address).

At the block 306 is represented the step wherein the user decodes the license key coded in the QR code and transmits the relative information to the management server. Thus, at block 306 the management server receives the license key by the user.

If the operation at the block 306 is successful, the management server checks if the license key received corresponds to the license key generated. If this operation is successful, the user is registered.

Instead of QR codes for representing the license key, a NFC device is used. The coded license key at the step 303 is stored in a NFC device. This is a NFC Tag and can be delivered in form of a small chip or visit card. For NFC usage the mobile device must have a NFC sensor or be able to extend with an external NFC sensor. At the step 304, the device NFC is assigned at the user. The user then retrieves the license key approximating his smartphone at the NFC device within its operative radius. The remaining operations are the same.

The registration process may comprise the further step of choosing a password by the user. At the end of registration process, the user is asked if he wants to choose a password. In this way a robust security check is carried out.

The information between the user equipment and the management server are exchanged as REST service calls.

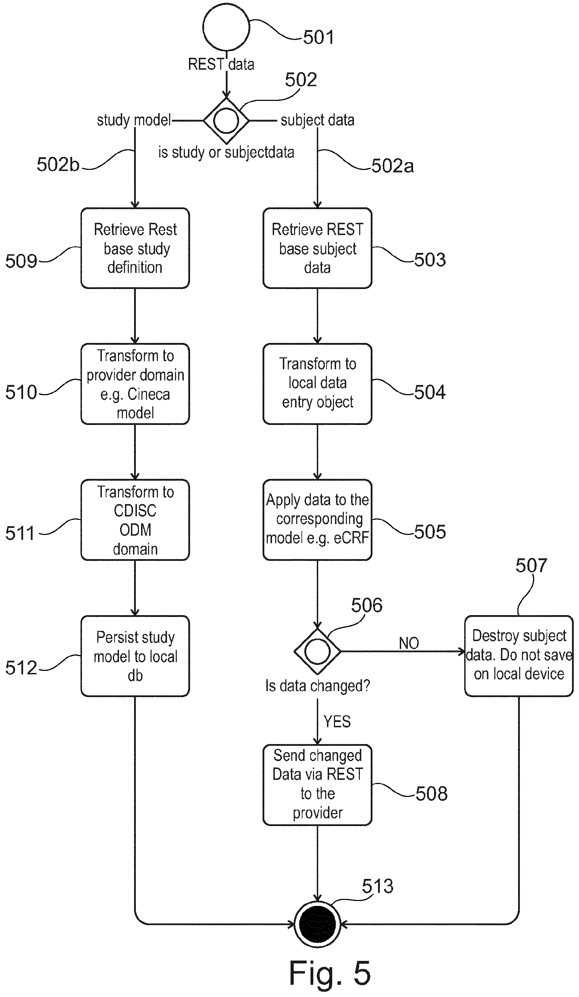

Figure 5 shows a block diagram of such an implementation.

At block 501 a REST data is received. Then the block 502 is reached. In this block such data is verified to assess if corresponds to a study model data or a subject data. If it is subject data branch 502a is followed and blocks from 503 to 508 are executed. In the block 503 the REST base subject data is retrieved. Then the block 504 is reached where such data is transformed in a local data entry object. Then the block 505 is reached where the data is applied to the corresponding model, for example the eCRF model. Then the test block 506 is reached where it is assessed if the data is changed. If the data is not changed the branch NO is followed, reaching the block 507 where the subject data is destroyed and it is not saved on the local device. Instead, if the data is changed, the branch YES is followed reaching the block 508 where the data are sent via REST to the provider. The blocks from 509 to 512 are executed if at the test block 502 it is verified that the REST data is a study model data, following the branch 502b for reaching the block 509 wherein the REST base study definition is retrieved. Then the block 510 is reached wherein the Study definition is transformed to provider domain, for example third party study model. The block 511 is then reached wherein the provider domain is transformed to CDISC ODM compatible domain. Then reaching the block 512 the study model is stored in the local database. This is only available if an offline mode is allowed. For online mode usage the model persist only in the memory of the mobile device and the information is lost after closing the application. The block 512, the block 507 and the block 508 end to the block 513.

The user may input the data when updating a case report form via voice commands. Voice commands are always additions to the standard touch events for navigating or inserting values with a virtual keyboard displays on the mobile device. Means if no VR is possible the standard Touch and Type event will work anyway. The user may give commands to the application, for example to navigate through case report forms, via voice commands as well.

At the management server a consistency check is carried out on the content of said one or more case report stored on the user equipment to synchronize with the content at said management server. In practice, when the management server has to synchronize the information of one or more case report form, check if the value stored are within acceptable ranges. For example, an acceptable range for the blood pressure may vary between 50/100 and 50/250 (systolic and diastolic values). Values outside this range may require a confirmation from the user before they are stored. Namely an on time eQuery.

The user equipment is typically a smartphone, or a tablet or customized mobile hardware for the purpose, the program is a computer program product conceived for mobile devices.

Further disclosed is a management server comprising at least a processing unit, memory means, data communication means and a program loaded in said memory means and adapted to be executed by said processing unit, said program when running configured to carry out the following steps:
- associating a user to a user equipment,
- authenticating said user,
- generating or retrieving one or more case report form associated to said user,
- delivering the generated or retrieved case report form to said user,
- receiving one or more updated case report form from said user,
- synchronizing the content of the one or more updated case report form received with the corresponding one or more case report form associated to said user.

The program stored on and executed by the management server is further configured to carry out the following steps:
- generating a unique license key,
- detecting a unique identifier of a user equipment,
- registering a user associated to a user equipment.

The program stored on and executed by the management server is further configured to carry out a consistency check before synchronizing the content of the one or more updated case report form received with the corresponding one or more case report form associated to said user.

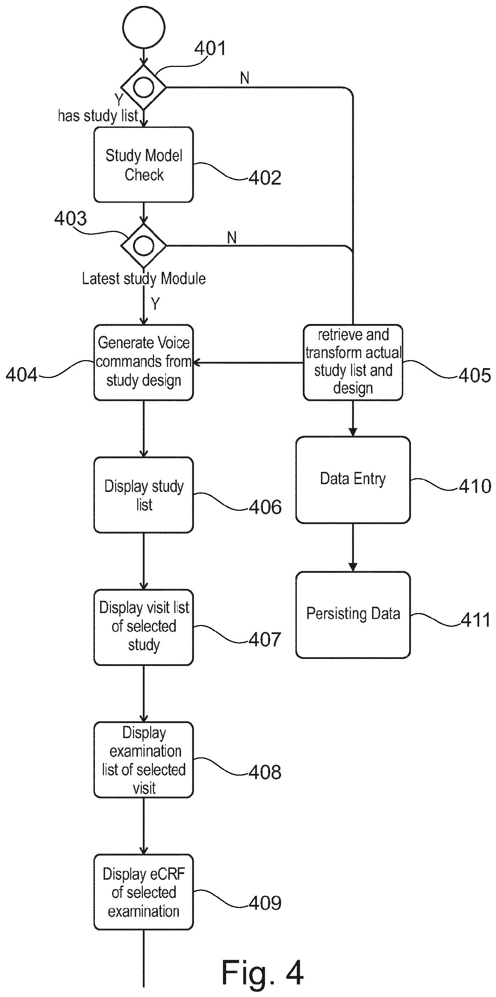

Figure 4 shows a block diagram representing the life cycle of
a computer program product implementing the method according the present invention. The first block 401 verify whether a study list is loaded on the user equipment. If it is loaded, the branch Y is followed, the study model is checked as shown in the block 402. If it is not loaded, the branch N is followed, the study model is retrieved as shown in the block 405. After the block 402, there is a further check, as shown in the block 403, to verify if the study loaded is the last group of studies actually conducted. If the result of this check is positive, voice commands are generated to retrieve a certain study. If the result of the check is negative, the branch N is followed and the block 405 is reached again, the study model is retrieved. The study list is displayed through the user equipment as shown in the block 406. On the basis of the selection made in the block 404, the selected study is displayed through the user equipment, as shown in the block 407. In one embodiment, the examination list of the selected visit is displayed, as shown in the block 408. In one embodiment, the electronic CRF of examination list of the selected visit is displayed, as shown in the block 409. At this point, the user (typically a medical practitioner) may affect the data entry on the basis of his observation, through the user equipment, as shown in the block 410. The data collected are then saved, as shown in the block 411.

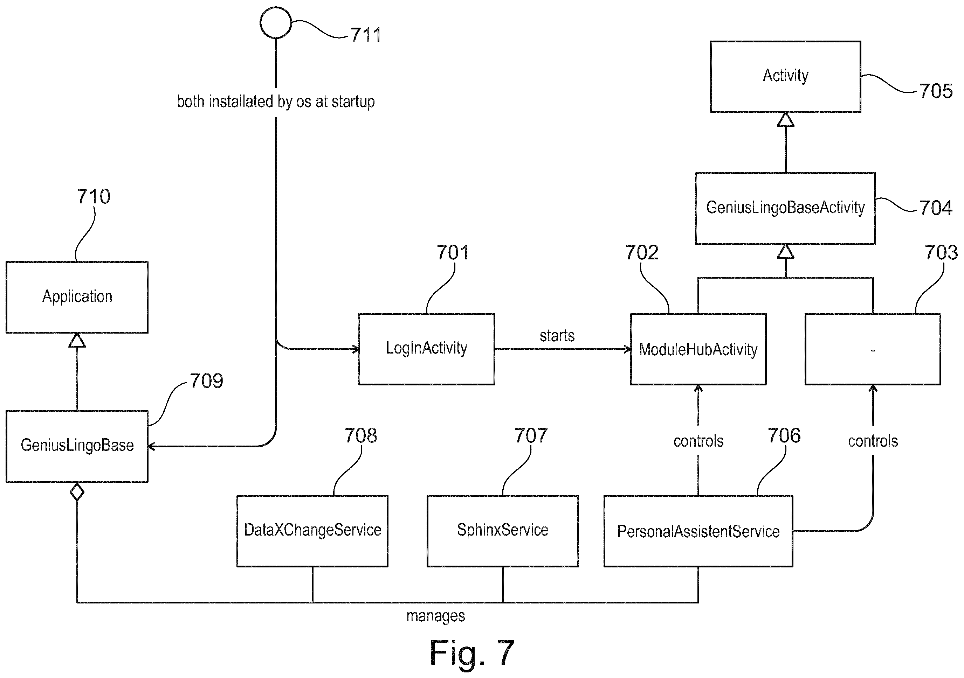

Figure 7 shows a block diagram representing the architecture of the present invention. In particular, activity is based on app navigation, not on fragments. Application class LaunchApp now maintains and manages the creation and destruction of services, control flow is solely based on decisions by the state-machine found in the PersonalAssistantService, i.e. the module represented in the block 706.

Block 711 represents the launch of the application, namely the execution of the method according the present invention. Block 701 represents the login activity. Block 702 represents the Module Hub activity. Block 704 is the GeniusLingoBase Activity, block 705 is the Activity. Block 708 is the DataXChange Service. Block 707 is the Sphinx Service. Block 706 is the Personal Assistant Service. Block 709 is the Genius Lingo Base. Block 710 is the Application.

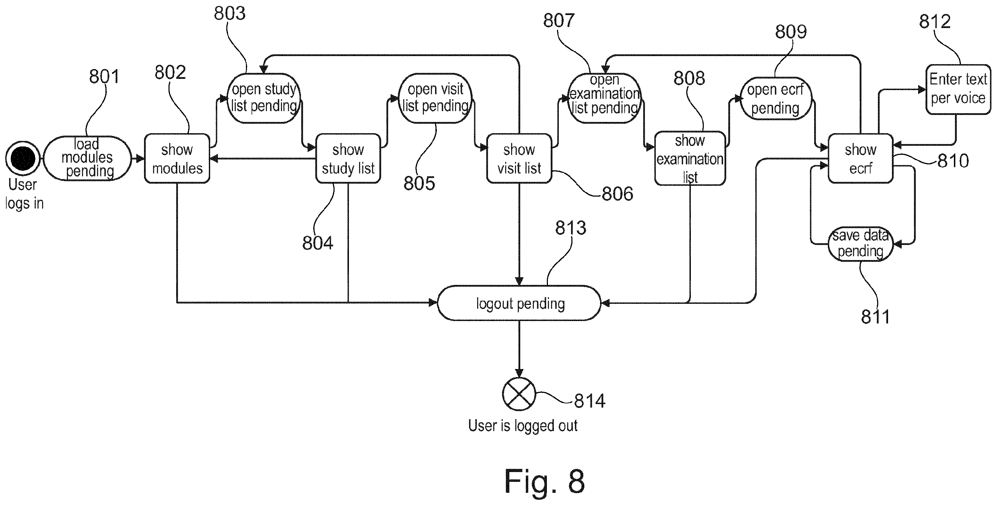

Figure 8 shows a block diagram representing the flows executed in the context of the present invention. Block 801 represents the load of the modules pending. Block 802 represents the operation of showing the modules loaded. Block 803 represents the open study list pending. Block 804 represents the operation of showing the study list subsequent. The block 804 points back to the block 802 to show the modules loaded and point to the block 805 to open visit list pending. The block 806 represents the operation of showing the visit list selected. The block 807 represents the operation of opening of the examination list pending. The block 808 represents the operation of showing the examination list. The block 809 represents the operation of opening the *ecrf* pending. The block 812 represents the operation of entering text per voice. The block 810 may alternatively ends up in the block 811 to save data pending. Blocks 802, 804, 806, 808, 810 may further end up in block 813 representing the logout pending. In turn the block 814 is reached, representing the user logged out. This is a standard workflow to iterate through the study model until data entry. The invention allows to adopt other workflow designs. Means the application is more dynamic. Further workflows are drug supply (e.g. new modul) or eQeuery or Annotation workflows within each step of Figure 8.

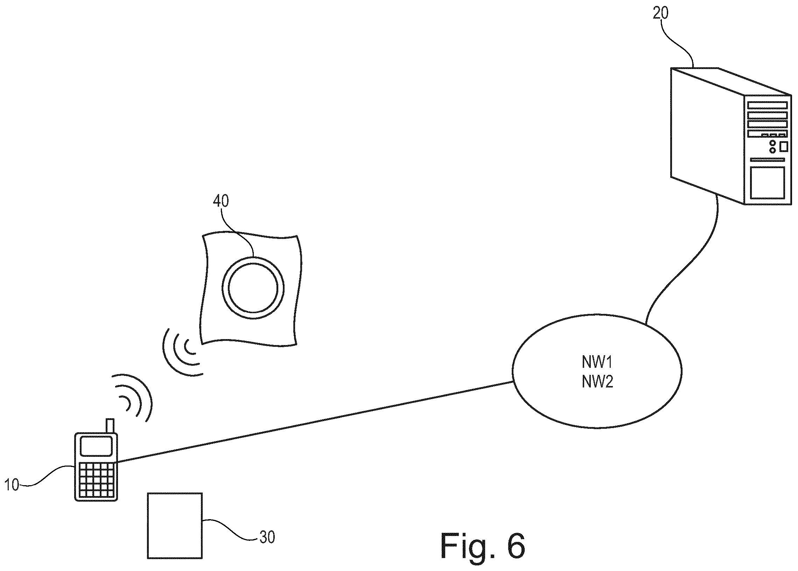

Figure 6 shows a diagram regarding synchronization of a clinical trials data implementing a method according the present invention.

As shown in figure 6, according to present invention further relates to a system for collecting and handling clinical trials data.

The system comprises
- at least a user equipment associated to a user as described above;
- at least a management server as described above,
- a telecommunication network NW1, NW2 to which the user through his user equipment and said management server are connected,
- a security device 30, 40 to register the user at the management server 20.

The network NW1 is typically a mobile network to which the user equipment is connected, the network NW2 is typically a fixed network to which the management server is connected.

The security device is a QR code 30, configured as described above or a NFC tag configure as described above.

## Claims

1. Method for collecting and handling clinical trials data wherein is provided a user associated to a user equipment and a management server comprising the following steps:
A - establishing a communication session between said user equipment and management server;
B - at said management server, authenticating said user;
C - at said management server, generating or retrieving one or more case report form associated to said user, said case report form comprising a plurality of information relative to a clinical trial;
D - delivering the one or more case report form generated or retrieved to said user via said user equipment,
E - at said user equipment, updating and/or viewing information of said one or more case report form,
F - at said user equipment, storing said one or more case report updated;
G - at said management server, synchronizing the content of said one or more case report form associated to said user with the content of the ones stored on the user equipment, **characterized in that** the step B of authenticating said user comprises a preliminary phase of registration of a user, made once for each user, comprising the following steps:
- at said management server, generating a unique license key,
- record said license number in a NFC tag,
- assigning said NFC tag to said user,
- establishing a communication between said user equipment and said management server,
- at said management server, detecting a unique identifier of said user equipment,
- via said user equipment retrieving the license key stored in said NFC tag,
- transmitting said license number to said management server,
- at said management server registering said user.

2. Method according to claim 1 wherein the preliminary phase of registration of said user comprise the following steps:
- at said user equipment choosing a password,
- transmitting said password to said management server,
- at said management server set registration information as the combination of said unique license key, said unique identifier of said user equipment and said password.

3. Method according to any of the preceding claims wherein the update and/or the view of information of said one or more case report at said step E occur via voice commands by said user.

4. Method according to any of the preceding claims wherein a consistency check is carried out on the content of said one or more case report stored on the user equipment to synchronize with the content at said management server.

5. System for collecting and handling clinical trials data comprising:
- at least a user equipment associated to a user comprising at least a processing unit, memory means, data communication means, acquiring means and/or proximity communication means
- at least a management server comprising at least a processing unit, memory means, and data communication means,
- a program loaded in said memory means and adapted to be executed by said processing unit, said program when running is configured to carry out a method according to one of claims 1 to 4,
- a telecommunication network (NW1, NW2) to which said user equipment and said management server are connected,
- a security device (30, 40) to register said user at said management server (20).

## Patentansprüche

1. Verfahren zur Erfassung und Bearbeitung von Daten aus klinischen Versuchen, bei dem ein Benutzer, der einer Benutzerausrüstung zugeordnet ist, und ein Verwaltungsserver vorgesehen sind, das die folgenden Schritte umfasst:
A - Herstellen einer Kommunikationssitzung zwischen der Benutzerausrüstung und dem Verwaltungsserver;
B - Authentifizieren des Benutzers auf dem Verwaltungsserver;
C - Erzeugen oder Abrufen eines oder mehrerer Fallberichtsformulare, die dem Benutzer zugeordnet sind, auf dem Verwaltungsserver, wobei das Fallberichtsformular eine Vielzahl von Informationen zu dem klinischen Versuch umfasst;
D - Übermitteln des einen oder der mehreren erzeugten oder abgerufenen Fallberichtsformulare an den Benutzer über die Benutzerausrüstung,
E - Aktualisieren und/oder Anzeigen von Informationen des einen oder der mehreren Fallberichtsformulare auf der Benutzerausrüstung,
F - Speichern des einen oder der mehrerer aktualisierten Fallberichte auf der Benutzerausrüstung;
G - Synchronisieren des Inhalts des einen oder der mehreren Fallberichtsformulare, die dem Benutzer zugeordnet sind, auf dem Verwaltungsserver mit dem Inhalt der auf der Benutzerausrüstung gespeicherten Formulare, **dadurch gekennzeichnet, dass** der Schritt B der Authentifizierung des Benutzers eine vorläufige Phase der Benutzerregistrierung umfasst, die einmal für jeden Benutzer durchgeführt wird und die folgenden Schritte umfasst:
- Erzeugen eines eindeutigen Lizenzschlüssels auf dem Verwaltungsserver,
- Aufzeichnen der Lizenznummer in einem NFC-Tag,
- Zuweisen des NFC-Tags zu dem Benutzer,
- Herstellen einer Kommunikation zwischen der Benutzerausrüstung und dem Verwaltungsserver,
- Erkennen einer eindeutigen Kennung der Benutzerausrüstung auf dem Verwaltungsserver,
- Abrufen des im NFC-Tag gespeicherten Lizenzschlüssels über die Benutzerausrüstung,
- Übertragen der Lizenznummer an den Verwaltungsserver,
- Registrieren des Benutzers auf dem Verwaltungsserver.

2. Verfahren nach Anspruch 1, wobei die vorläufige Phase der Benutzerregistrierung die folgenden Schritte umfasst:
- Auswählen eines Passworts auf der Benutzerausrüstung,
- Übertragen des Passworts an den Verwaltungsserver,
- Festlegen der Registrierungsinformationen auf dem Verwaltungsserver als Kombination aus dem eindeutigen Lizenzschlüssel, der eindeutigen Kennung der Benutzerausrüstung und dem Passwort.

3. Verfahren nach einem der vorhergehenden Ansprüche, wobei die Aktualisierung und/oder die Anzeige der Informationen des einen oder der mehreren Fallberichte in Schritt E über Sprachbefehle von dem Benutzer erfolgen.

4. Verfahren nach einem der vorhergehenden Ansprüche, wobei eine Konsistenzprüfung des Inhalts des einen oder der mehreren auf der Benutzerausrüstung gespeicherte Fallberichte durchgeführt wird, um eine Synchronisierung mit dem Inhalt auf dem Verwaltungsserver durchzuführen.

5. System zur Erfassung und Bearbeitung von Daten aus klinischen Versuchen, umfassend:
- mindestens eine Benutzerausrüstung, die einem Benutzer zugeordnet ist und mindestens eine Verarbeitungseinheit, Speichermittel, Datenkommunikationsmittel, Erfassungsmittel und/oder Nahkommunikationsmittel umfasst,
- mindestens einen Verwaltungsserver, der mindestens eine Verarbeitungseinheit, Speichermittel und Datenkommunikationsmittel umfasst,
- ein in die Speichermittel geladenes und zur Ausführung durch die Verarbeitungseinheit geeignetes Programm, wobei das Programm bei Ausführung dazu konfiguriert ist, ein Verfahren nach einem der Ansprüche 1 bis 4 auszuführen,
- ein Telekommunikationsnetz (NW1, NW2), mit dem die Benutzerausrüstung und der Verwaltungsserver verbunden sind,
- eine Sicherheitsvorrichtung (30, 40) zum Registrieren des Benutzers auf dem Verwaltungsserver (20).

## Revendications

1. Procédé de collecte et de traitement de données d'essais cliniques, dans lequel est fourni un utilisateur associé à un équipement utilisateur et un serveur de gestion comprenant les étapes suivantes :
A - établir une session de communication entre ledit équipement utilisateur et le serveur de gestion ;
B - au niveau dudit serveur de gestion, authentifier ledit utilisateur ;
C - au niveau dudit serveur de gestion, générer ou récupérer un ou plusieurs formulaires de rapport de cas associés audit utilisateur, ledit formulaire de rapport de cas comprenant une pluralité d'informations relatives à un essai clinique ;
D - remettre l'un ou plusieurs formulaires de rapport de cas générés ou récupérés audit utilisateur via ledit équipement utilisateur,
E - au niveau dudit équipement utilisateur, mettre à jour et/ou visualiser des informations desdits un ou plusieurs formulaires de rapport de cas,
F - au niveau dudit équipement utilisateur, stocker lesdits un ou plusieurs rapports de cas mis à jour ;
G - au niveau dudit serveur de gestion, synchroniser le contenu desdits un ou plusieurs formulaires de rapport de cas associés audit utilisateur avec le contenu de ceux stockés sur l'équipement utilisateur, **caractérisé en ce que** l'étape B d'authentification dudit utilisateur comprend une phase préliminaire d'enregistrement d'un utilisateur, effectuée une fois pour chaque utilisateur, comprenant les étapes suivantes :
- au niveau dudit serveur de gestion, générer une clé de licence unique,
- enregistrer ledit numéro de licence dans une étiquette NFC,
- attribuer ladite étiquette NFC audit utilisateur,
- établir une communication entre ledit équipement utilisateur et ledit serveur de gestion,
- au niveau dudit serveur de gestion, détecter un identifiant unique dudit équipement utilisateur,
- via ledit équipement utilisateur récupérer la clé de licence stockée dans ladite étiquette NFC,
- transmettre ledit numéro de licence audit serveur de gestion,
- au niveau dudit serveur de gestion enregistrer ledit utilisateur.

2. Procédé selon la revendication 1, dans lequel la phase préliminaire d'enregistrement dudit utilisateur comprend les étapes suivantes :
- au niveau dudit équipement utilisateur choisir un mot de passe,
- transmettre ledit mot de passe audit serveur de gestion,
- au niveau dudit serveur de gestion, définir les informations d'enregistrement comme la combinaison de ladite clé de licence unique, dudit identifiant unique dudit équipement utilisateur et dudit mot de passe.

3. Procédé selon l'une quelconque des revendications précédentes, dans lequel la mise à jour et/ou la visualisation des informations desdits un ou plusieurs rapports de cas à ladite étape E se produisent via des commandes vocales par ledit utilisateur.

4. Procédé selon l'une quelconque des revendications précédentes, dans lequel un contrôle de cohérence est effectué sur le contenu desdits un ou plusieurs rapports de cas stockés sur l'équipement utilisateur pour se synchroniser avec le contenu au niveau dudit serveur de gestion.

5. Système de collecte et de traitement des données d'essais cliniques comprenant :
- au moins un équipement utilisateur associé à un utilisateur comprenant au moins une unité de traitement, des moyens de mémoire, des moyens de communication de données, des moyens d'acquisition et/ou des moyens de communication de proximité,
- au moins un serveur de gestion comprenant au moins une unité de traitement, des moyens de mémoire et des moyens de communication de données,
- un programme chargé dans lesdits moyens de mémoire et adapté pour être exécuté par ladite unité de traitement, ledit programme en cours d'exécution étant configuré pour mettre en œuvre un procédé selon l'une des revendications 1 à 4,
- un réseau de télécommunication (NW1, NW2) auquel sont connectés ledit équipement utilisateur et ledit serveur de gestion,
- un dispositif de sécurité (30, 40) pour enregistrer ledit utilisateur sur ledit serveur de gestion (20).
